(19) Europäisches Patentamt / European Patent Office / Office européen des brevets

(11) **EP 3 897 674 B1**

(12) **EUROPEAN PATENT SPECIFICATION**

(45) Date of publication and mention of the grant of the patent:
**06.09.2023 Bulletin 2023/36**

(21) Application number: **18847149.4**

(22) Date of filing: **19.12.2018**

(51) International Patent Classification (IPC):
*A61K 31/765* (2006.01)   *A61P 1/02* (2006.01)
*A61P 31/04* (2006.01)   *A61P 31/10* (2006.01)
*A61P 9/00* (2006.01)   *A61Q 11/00* (2006.01)

(52) Cooperative Patent Classification (CPC):
**A61K 31/765; A61P 1/02; A61P 9/00; A61P 31/04; A61P 31/10**

(86) International application number:
**PCT/IB2018/060366**

(87) International publication number:
**WO 2020/128584 (25.06.2020 Gazette 2020/26)**

(54) **POLYHYDROXYALKANOATE (PHA) FOR USE IN THE TREATMENT AND/OR PREVENTION OF ORAL CAVITY DISEASES**

POLYHYDROXYALKANOAT (PHA) ZUR VERWENDUNG IN DER BEHANDLUNG UND/ODER VORBEUGUNG VON ERKRANKUNGEN DER MUNDHÖHLE

POLYHYDROXYALKANOATE (PHA) À UTILISER DANS LE TRAITEMENT ET/OU LA PRÉVENTION DE MALADIES DE LA CAVITÉ BUCCALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**27.10.2021 Bulletin 2021/43**

(73) Proprietor: **Bio-On S.p.A.**
**40016 San Giorgio Di Piano (BO) (IT)**

(72) Inventors:
• **SAETTONE, Paolo**
**40016 San Giorgio Di Piano (BO) (IT)**
• **CONDO', Carla**
**40016 San Giorgio Di Piano (BO) (IT)**
• **CELLANTE, Lina**
**40016 San Giorgio Di Piano (BO) (IT)**
• **CICOGNANI, Veronica**
**40016 San Giorgio Di Piano (BO) (IT)**
• **ALIFANO, Pietro**
**73100 Monteroni (LE) (IT)**
• **CALCAGNILE, Matteo**
**73100 Monteroni (LE) (IT)**
• **COMES FRANCHINI, Mauro**
**40016 San Giorgio Di Piano (BO) (IT)**

(74) Representative: **Bottero, Carlo et al**
**Barzanò & Zanardo Milano S.p.A.**
**Via Borgonuovo, 10**
**20121 Milano (IT)**

(56) References cited:
**EP-A1- 1 797 858**      **WO-A1-2020/003091**
**WO-A2-2007/146173**

• **KARAHALILOGLU ZEYNEP ET AL: "Antibacterial Nanostructured Polyhydroxybutyrate Membranes for Guided Bone Regeneration.", JOURNAL OF BIOMEDICAL NANOTECHNOLOGY DEC 2015, vol. 11, no. 12, December 2015 (2015-12), pages 2253-2263, XP9515363, ISSN: 1550-7033**
• **VON SCHWANEWEDE H ET AL: "Optimization of the neck region of dental implants with a bioactive, resorbable coating.", BIOMEDIZINISCHE TECHNIK. BIOMEDICAL ENGINEERING 2002, vol. 47 Suppl 1 Pt 1, 2002, pages 479-481, XP9515366, ISSN: 0013-5585**

- SENDIL D ET AL: "Antibiotic release from biodegradable PHBV microparticles", JOURNAL OF CONTROLLED RELEASE, ELSEVIER, AMSTERDAM, NL, vol. 59, no. 2, 20 May 1999 (1999-05-20), pages 207-217, XP004169750, ISSN: 0168-3659, DOI: 10.1016/S0168-3659(98)00195-3
- REUSCH ROSETTA N: "Poly-(R)-3-hydroxybutyrates (PHB) are atherogenic components of lipoprotein Lp(a)", MEDICAL HYPOTHESES, vol. 85, no. 6, December 2015 (2015-12), pages 1041-1043, XP002793628, ISSN: 0306-9877

**Description**

The present invention relates to a polyhydroxyalkanoate (PHA) for use in the treatment and/or prevention of oral cavity diseases, as defined in the claims. In particular, the present invention relates to a polyhydroxyalkanoate (PHA) for use in the treatment and/or prevention of oral cavity diseases, through the sequestration of pathogenic microorganisms by the PHA and consequent removal of the same from the dental surface.

The references to methods of treatment in this description are to be interpreted as references to the compounds, pharmaceutical compositions and medicaments of the present invention for use in a method for treatment of the human (or animal) body by therapy.

It is well known that the oral cavity constitutes an ecosystem formed by different habitats, and has a high dynamicity due to the continuous elimination and introduction of microorganisms and food. It is colonized by hundreds of microbial species that occupy specialized niches forming more or less complex biofilms. The resident microbial population is of great biodiversity, justified by the different habitats and the variety of nutritious materials. In many cases, these are potentially pathogenic bacterial microorganisms that can cause morbid events (also of a considerable entity) if they manage to access deep tissues, or the bloodstream, through traumas and surgical wounds. Over 350 different microbial species permanently live in the oral cavity. The dominant species are staphylococci, streptococci and actinomycetes. The development of the oral cavity microbiota is strongly influenced by streptococci, which constitute the main group of precocious colonizers, their presence determines the composition of the late colonizers of the oral biofilm and influences the state of health or illness of the host. For example, *Streptococcus salivarius* is one of the earliest colonizers of the oral cavity and seems to actively contribute to the establishment of the host's immune homoeostasis.

The oral cavity mainly comprises the lips, cheeks, palate, teeth and gingival sulcus. The teeth have a hard surface which, unlike many other body surfaces, is not subject to rapid regeneration. This is an important aspect, since many bacteria that have the possibility to adhere to this surface can only be removed mechanically.

The possibility for bacteria to adhere to this surface is determined by so-called aggregating factors, i.e. compounds, for example glycoproteins, which facilitate the aggregation of bacteria. The aggregating factors are extremely important because the coaggregated bacterial masses in the oral cavity which then adhere to the dental surface are the first step in the formation of plaque and dental caries.

Dental caries is a multi-factor disease with a bacterial aetiology, with a complex pathogenesis. It is defined as a "conditional" disease, that is, only the occurrence of certain conditions allows the potential pathogen to fully manifest itself.

The disease affects the hard tissues of the tooth, caused by endogenous microorganisms such as *Streptococcus mutans* and *Streptococcus sobrinus,* characterized by the demineralization of the enamel and the subsequent alteration of the dentin with final colonization of the dental pulp. The starting point of the pathological process is represented by the formation of plaque composed of bacteria, salivary organic materials and extracellular microbial products that create a complex biofilm which is firmly attached to the enamel surface and whose presence subjects the dental and gingival tissues to possible damage.

In addition to the microorganisms mentioned above, the extensive resident flora of the oral cavity also includes *Streptococcus sanguis* which, although not involved in dental caries, represents an aggressive opportunistic pathogen responsible for infective endocarditis in predisposed subjects.

As described above, since the bacteria that have the possibility of adhering to the dental surface can only be removed mechanically and, inside the oral cavity, the bacterial masses coaggregated and then adhered to the dental surface represent the first step for the formation of plaque and dental caries, in order to prevent and/or cure many oral cavity diseases it is important to interact specifically with these cariogenic bacterial masses in order to remove them from the dental surface.

To date, treatments for the prevention and/or cure of oral cavity diseases, wherein the aggregation and adhesion of pathogenic bacteria to the dental surface plays a crucial role, are based on the mechanical removal of the bacterial aggregates that have already adhered to the dental surface, such as the mechanical removal of plaque by tartar ablation or scaling.

The international application WO2007/146173 describes a method for treating or preventing caries by applying a polymer, to which caries-causing bacteria adhere and then are removed.

The Applicant has therefore posed the problem of identifying a non-invasive method for the prevention and/or cure of oral cavity diseases which favours the specific removal of bacteria adhering to the dental surface which are responsible for the aforesaid diseases.

The Applicant has now found that this problem, and others that will be better illustrated hereinafter, can be solved by using a polyhydroxyalkanoate (PHA), which when introduced into the oral cavity, while not presenting inhibitory activity against the growth of cariogenic streptococci (such as *S. mutans* and *S. sobrinus*), opportunistic pathogens (*S. Sanguis*) and non-cariogenic pathogens (for example *S. salivarius*), is able to coaggregate (in a more or less marked way according to the bacterial strain) such microorganisms on itself, thus removing them from the interaction with the dental surface and the mucous membranes of the mouth. This property, which can be compared to a sort of "magnetic effect" by the PHA on microorganisms, can be usefully exploited since the PHA, on whose surface the coaggregation of microorganisms occurs quickly, can be easily removed, significantly reducing the bacterial load of the oral cavity, in particular the load of cariogenic and/or opportunist microorganisms responsible for many oral cavity diseases.

The present invention therefore describes but does not claim a polyhydroxyalkanoate (PHA) for use in the treatment and/or prevention of diseases caused by pathogenic microorganisms of the oral cavity. More specifically, the aforesaid use is carried out through the sequestration of pathogenic microorganisms by the PHA and consequent removal of the same from the dental surface.

In the context of the present description and the attached claims, the term "sequestration" refers to the ability of a substance to bind to itself, and therefore subtract one or more biological entities from the surrounding environment.

In the context of the present description and the attached claims, the term "coaggregation" refers to a typical phenomenon of the oral ecosystem where bacteria benefit from adhering to other microorganisms by exploiting different bridge structures or molecules. The following are, for non-limiting purposes, some examples of bridge structures or molecules used by bacteria for coaggregation:

- coaggregation mediated by molecules such as glucan and glycoproteins;
- direct coaggregation between bacteria, mediated by molecules present on the external bacterial surface (afimbrial adhesins) or bacterial appendages (fimbriae).

In the context of the present description and the attached claims, the term "adhesion to the dental surface" refers to the ability of a microorganism, in particular a pathogenic microorganism, to bind to the dental surface, which is the first step leading to the colonization of the surface by the microorganism itself.

Polyhydroxyalkanoates (PHA) are polymers produced by microorganisms isolated from natural environments or even by genetically modified microorganisms, and are characterized by a high biodegradability. They are produced and accumulated by various species of bacteria under unfavourable growth conditions and in the presence of a source of excess carbon. PHAs are synthesized and accumulated by about 300 different microbial species, included in more than 90 kinds of Gram-positive and Gram-negative bacteria, such as for example *Bacillus, Rhodococcus, Rhodospirillum, Pseudomonas, Alcaligenes, Azotobacter, Rhizobium.*

In bacterial cells, PHA is stored in the form of microgranules, whose size and number per bacterial cell varies in different species. They appear as refractive inclusions under an electron microscope, with a diameter comprised from 0.2 to 0.7 um.

As for the PHA, this is preferably a polymer containing repeating units of formula:

$$-O-CHR_1-(CH_2)_n-CO- \qquad (I)$$

where:

$R_1$ is selected from: $C_1$-$C_{12}$ alkyls, $C_4$-$C_{16}$ cycloalkyls, $C_2$-$C_{12}$ alkenyls, optionally substituted with at least one group selected from: halogen (F, Cl, Br), -CN, -OH, -OOH, -OR, -COOR (R = $C_1$-$C_4$ alkyl, benzyl);
n is zero or an integer from 1 to 6, preferably is 1 or 2.

Preferably, $R_1$ is methyl or ethyl, and n is 1 or 2.

The PHAs can either be homopolymers, copolymers or terpolymers. In the case of copolymers or terpolymers, they can consist of different repeating units of formula (I), or of at least one repetitive unit of formula (I) in combination with at least one repetitive unit deriving from comonomers that are able to copolymerize with hydroxyalkanoates, such as lactones or lactams. In the latter case, the repeating units of formula (I) are present in an amount equal to at least 10% in moles with respect to the total moles of repetitive units.

Particularly preferred repeating units of formula (I) are those deriving from: 3-hydroxybutyrate, 3-hydroxyvalerate, 3-hydroxyhexanoate, 3-hydroxyoctanoate, 3-hydroxyundec-10-enoate, 4-hydroxyvalerate.

Particularly preferred PHAs are: polyhydroxybutyrate (PHB), poly-3-hydroxyvalerate (PHV), poly-3-hydroxyhexanoate (PHH), poly-3-hydroxyoctanoate (PHO), poly(3-hydroxybutyrate-co-3-hydroxyvalerate) (PHBV), poly(3-hydroxybutyrate-co-3-hydroxyhexanoate) (PHBH), poly(3-hydroxybutyrate-co-4-hydroxybutyrate), poly(3-hydroxyoctanoate-co-3-hydroxyundecen-10-enoate) (PHOU), poly(3-hydroxybutyrate-co-3-hydroxyvalerateco-4-hydroxyvalerate) (PHBW), polyhydroxybutyrate-hydroxyvalerate copolymer, or mixtures thereof.

According to the purposes of the present invention, a particularly preferred PHA is polyhydroxybutyrate (PHB).

PHAs suitable for the present invention preferably have a weight average molecular weight (Mw) comprised in the range from 5,000 to 1,500,000 Da, more preferably from 100,000 to 1,000,000 Da. The weight average molecular weight can be determined according to known techniques, in particular by means of GPC analysis (Gel Permeation Chromatography).

As for the production of PHA, it is preferably obtained from the microbial fermentation of an organic substrate (for example carbohydrates or other fermentable substrates, such as glycerol) by means of a strain of microorganisms capable of producing PHA, and the subsequent recovery of the PHAs from the cell mass.

For further details, reference can be made, for example, to patent applications WO 99/23146, WO 2011/045625 and WO 2015/015315. Suitable substrates for the production of PHAs by fermentation can be obtained in particular from the processing of vegetables, for example juices, molasses, pulps from the processing of sugar beet, sugar cane.

These substrates generally contain, in addition to sucrose and other carbohydrates, organic growth factors, nitrogen, phosphorus and/or other minerals which are useful as nutrients for cell growth.

An alternative is glycerol, a low-cost organic carbon source, as it is a by-product of biodiesel production (see, for example, US patent 8 956 835 B2).

According to the purposes of the present invention, the diseases caused by pathogenic microorganisms of the oral cavity are in particular typical oral cavity diseases, such as: caries, gingivitis, periodontitis, perimplantitis, halitosis.

Moreover, the diseases caused by pathogenic microorganisms of the oral cavity are diseases of organs other than the oral cavity, which are caused by the penetration of these microorganisms into the blood flow, so as to reach and infect other organs of the body. Examples of such diseases are: bacteraemia, sepsis, infective endocarditis and pericarditis, development of atheromatous plaques.

Preferably, pathogenic microorganisms or opportunistic pathogens on which PHAs can advantageously intervene in accordance with the present invention are for example: *Streptococcus mutans, Streptococcus sobrinus, Streptococcus sanguis, Porfiromonas gingivalis, Aggregatibacter actinomycetemcomitans,* or fungi such as *Aspergillus fumigatus, Aspergillus flavus* and *Candida albicans.*

Surprisingly, from the preliminary studies conducted by the Applicant (described in detail below) concerning the *in vitro* interaction between a PHA and certain microorganisms of the oral cavity, an evident ability of the PHA to attract these microorganisms emerged, which are coaggregated on the surface of the polymer and are thus subtracted and isolated from the surrounding environment. This occurs relatively quickly (from one to a few minutes). Thus, the PHA can easily be removed from the oral cavity together with the microorganisms which have adhered to its surface, for example by rinsing with water, physiological solution or mouthwash.

This capacity can therefore be exploited to create oral cavity care formulations (the so called "oral care formulations"), in order to promote the removal of the bacterial film from the dental surface. One of the purposes of these formulations is indeed to inhibit the accumulation of dental plaque through the removal of cariogenic bacteria or responsible for periodontal diseases.

Therefore, the present invention relates to a formulation comprising at least one polyhydroxyalkanoate (PHA) as defined above and at least one physiologically acceptable excipient, for use in the treatment and/or prevention of diseases caused by pathogenic microorganisms of the oral cavity

wherein the PHA is polyhydroxybutyrate (PHB) in the form of particles with an average diameter (d50) from 0.1 um to 500 um;
wherein the diseases caused by pathogenic microorganisms of the oral cavity are selected from: caries, gingivitis, periodontitis, perimplantitis, halitosis,
wherein the formulation is selected from: toothpaste, chewing gum, mouthwash, dental paste, orodispersible tablet, gingival dye.

The present invention describes but does not claim a method for treating and/or preventing diseases caused by pathogenic microorganisms of the oral cavity, which comprises:

applying within the oral cavity a formulation comprising at least one polyhydroxyalkanoate (PHA) and at least one physiologically acceptable excipient,
maintaining said formulation within the oral cavity for a sufficient amount of time to obtain the coaggregation of said pathogenic microorganisms on the surface of the PHA;
removing said PHA from the oral cavity on which said microorganisms have coaggregated.

On the basis of the experiment carried out by the Applicant, the time needed to obtain the coaggregation of said pathogenic microorganisms on the surface of the PHA can vary within wide limits, generally from 30 seconds to 30 minutes, preferably from 1 minute to 15 minutes. The time can vary depending on various factors, such as the condition of the oral cavity, in particular the extent of infection by pathogenic microorganisms, the amount of PHA present in the formulation, the nature of the pathogenic microorganisms present, etc.

In the context of the present description and the attached claims, the term "physiologically acceptable excipient" refers to a substance lacking significant effects from the physiological point of view, which gives to a formulation the form, consistency, dilution and other physical and chemical-physical characteristics required, mainly acting as a vehicle for the active substances.

The PHA is in the form of particles with an average diameter (d50) from 0.1 um to 500 $\mu$m, preferably from 0.5 um to 300 $\mu$m, more preferably from 1 um to 100 um.

The PHA particles are preferably characterized by high porosity, with a surface area value (BET) preferably from 0.5 to 20 m²/g, more preferably from 1 to 10 m²/g, measured according to norm ISO 9277:1995.

Preferably, the PHA particles are obtained through an atomization process, through spray drying of an aqueous PHA suspension.

In the context of the present description and the attached claims, "average particle diameter" refers to, unless otherwise indicated, the diameter d50 (median value), i.e. the value of the diameter below which there is 50% by weight of the particle population (see "A Guidebook to Particle Size Analysis" published by Horiba Instruments Inc. - 2016, available on https://www.horiba.com/fileadmin/uploads/Scientific/ eMag/PSA/Guidebook/pdf/PSA_Guidebook.pdf). It can be determined through a laser diffraction technique, according to norm ISO 13320:2009.

The PHA is preferably present in the formulation in a concentration comprised from 1% to 30%, more preferably from 2% to 15% by weight, the % being expressed with respect to the overall weight of the formulation.

According to the present invention, the formulation is selected from: toothpaste, chewing gum, mouthwash, dental paste (to be applied on the inner surface of a dental "bite", for example), orodispersible tablet, and gingival dye.

Depending on the specific formulation, at least one excipient can be selected from the ingredients commonly used in the field of dental hygiene products, such as: humectants (for example sorbitol, xylitol, glycerin, polyethylene glycol, hydrogenated starch hydrolyzate (HSI)), abrasives (for example silica or calcium carbonate), thickening agents (for example cellulose or its derivatives, such as cellulose gum), detergents (for example surfactants such as sodium lauryl sulphate), flavourings, anti-plaque agents (in particular bactericides such as zinc or tin salts, chlorhexidine), anti-caries agents (for example fluoride salts), anti-tartar agents (such as potassium or sodium pyrophosphates), preservatives, dyes, soothing agents (for example bisabolol), emollients (for example strontium chloride), whiteners (for example papain), emulsifiers (for example lecithin), plasticizers, fillers (for example waxes), elastomers (for example natural rubber). The formulation also generally contains water, in order to solubilize or disperse the components, in an amount such as to obtain the desired fluidity.

The following embodiment examples are provided for the sole purpose of illustrating the present invention and are not to be understood in a sense limiting the scope of protection defined by the appended claims.

EXAMPLE 1: Evaluation of the microbial coaggregation kinetics on particles of PHB (polyhydroxybutyrate).

The evaluation of the microbial coaggregation kinetics on PHB particles was performed using the following bacterial strains: *S. mutans* ATCC 25175 (cariogenic), *S. sobrinus* ATCC 33478 (cariogenic); *S. salivarius* ATCC 13419 (non-cariogenic) e *S. sanguis* ATCC 10556 (opportunistic pathogen). The PHB had the following characteristics: weight average molecular weight (Mw) equal to 400,000; average diameter (d50) of the particles equal to 20 um.

The tests were carried out in triplicate for each microbial species, dispersing the PHB in a 10% dimethylsulfoxide (DMSO) saline solution at five different concentrations: 10, 17, 50, 100 and 150 mg/mL. The test was conducted for a total time of 30 minutes, determining the interaction kinetics at different time intervals (10 seconds, 2, 4, 6, 8, 10, 15, 20, 25 and 30 minutes), by drawing 10 $\mu$L of suspension, diluted up to the concentration of PHB equal to 10 mg/mL. After deposition on a slide, the coaggregation of the bacteria on the PHB particles was evaluated through optical microscopy (100x magnification), after fixation and staining with a Gram kit.

The extent of the coaggregation was quantified using the following parameter:

$$r = n/m$$

where: n is the coaggregated bacteria count on the PHB particles; m is the number of PHB particles that have bacteria adhering to them. The count was performed in 20 different regions of the sample surface, using the freeware Image J (NIH, Bethesda MD).

The microorganisms in 10% saline solution of DMSO, without the addition of PHB, were used as a negative control.

The coaggregation of the microbial strains on the PHB particles was rather rapid, taking only a few minutes. At the lowest concentrations tested (10 and 17 mg/mL) no difference in effect was found: the number of bacteria per PHB particle was, in fact, comparable. At higher concentrations (50, 100 and 150 mg/mL), however, greater number of bacterial aggregates were found on the PHB particles.

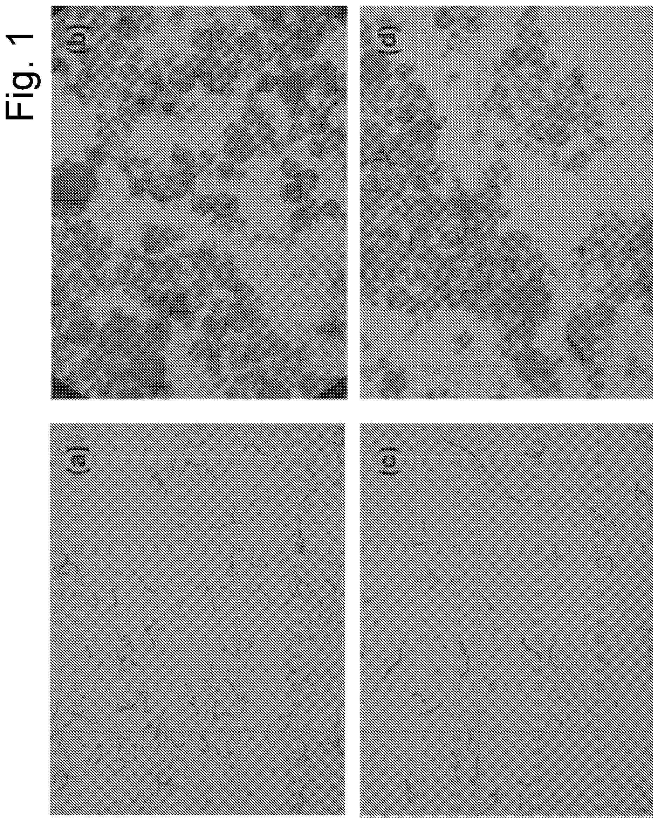

Figure 1 shows some photographs taken with the microscope (optical images with 100X magnification) related to the tests with *S. mutans* ATCC 25175 (images (a) and (b)) and with *S. sobrinus* ATCC 33478 (images (c) and (d)).

Images (a) and (c) are the negative controls (without PHB), while images (b) and (d) are relative to samples containing PHB after 1 minute of contact with the bacterial culture. The PHB particles (dark grey colour) on which the bacterial colonies (black dashes) have adhered can be noted.

The ratio r between n (number of bacteria coaggregated on the PHA surface) and m (PHA particles presenting coaggregated bacteria) tends to increase with time, for all the concentrations considered. After 8 minutes, a plateau phase was observed, wherein the strains analysed showed comparable results. S. *salivarius* showed values of the r parameter at the lowest contact times, lower than the other strains, while *S. sanguis, S. mutans* and *S. sobrinus* showed very similar trends, among them, over time.

Table 1 shows the values of r (average values on the three samples analysed, with relative standard deviation, STD) obtained for *S. sanguis* and *S. salivarius,* at the concentration of 10 mg/mL of PHB.

TABLE 1

| Time | *S. sanguis* ATCC 10556 | | *S. salivarius* ATCC 13419 | |
| --- | --- | --- | --- | --- |
| | Parameter r | STD | Parameter r | STD |
| 10 sec | 2.01 | 0.08 | 1.66 | 0.10 |
| 2 min | 2.16 | 0.04 | 1.84 | 0.07 |
| 4 min | 2.23 | 0.17 | 2.34 | 0.10 |
| 6 min | 2.55 | 0.09 | 2.33 | 0.09 |
| 8 min | 2.56 | 0.12 | 2 . 65 | 0.11 |
| 10 min | 2.64 | 0.08 | 2 . 65 | 0.10 |
| 15 min | 2.67 | 0.08 | 2.71 | 0.11 |
| 20 min | 2.77 | 0.10 | 2.78 | 0.14 |
| 25 min | 2.84 | 0.08 | 2.90 | 0.11 |
| 30 min | 2.88 | 0.03 | 2.89 | 0.12 |

**EXAMPLE 2: toothpaste formulation.**

a) Four different toothpaste formulations were made, each containing 5% or 15% of PHB: standard formulation, sensitive formulation, that is for sensitive teeth, whitening formulation, that is with a whitening effect, and kids formulation for children from 0 to 6 years. Tables 2, 3, 4 and 5 show the qualitative composition (*International Nomenclature of Cosmetic Ingredients* - INCI) of each formulation.

TABLE 2 - standard formulation

| Ingredient | Function |
| --- | --- |
| Water | / |
| Hydrogenated starch hydrolyzate (HSI) | Humectant |
| Hydrated silica | Abrasive |
| PEG-32 | Humectant |
| Cellulose | Thickening agent |
| Sodium saccharin | Sweetener |
| Sodium fluoride | Anti-caries agent |
| Zinc citrate | Anti-plaque agent |
| Flavouring | / |
| Oil of *Thymus vulgaris* leaves | Preservative |
| CI-77891 | Dye |

(continued)

| Ingredient | Function |
| --- | --- |
| PHB | |

TABLE 3 - sensitive formulation

| Ingredient | Function |
| --- | --- |
| Water | / |
| Hydrogenated starch hydrolyzate (HSI) | Humectant |
| Hydrated silica | Abrasive |
| PEG-32 | Humectant |
| Cellulose | Thickening agent |
| Sodium saccharin | Sweetener |
| Bisabolol | Soothing agent |
| Sodium fluoride | Anti-caries agent |
| Zinc citrate | Anti-plaque agent |
| Strontium chloride | Emollient |
| Flavouring | / |
| Oil of *Thymus vulgaris* leaves | Preservative |
| CI-77891 | Dye |
| PHB | |

TABLE 4 - whitening formulation

| Ingredient | Function |
| --- | --- |
| Water | / |
| Hydrogenated starch hydrolyzate (HSI) | Humectant |
| Hydrated silica | Abrasive |
| PEG-32 | Humectant |
| Cellulose | Thickening agent |
| Sodium saccharin | Sweetener |
| Sodium fluoride | Anti-caries agent |
| Papain | Whitener |
| Zinc citrate | Anti-plaque agent |
| Flavouring | / |
| Oil of *Thymus vulgaris* leaves | Preservative |
| CI-77891 | Dye |
| CI-74160 | Dye |
| PHB | |

TABLE 5 - kids formulation

| Ingredient | Function |
|---|---|
| Water | / |
| Hydrogenated starch hydrolyzate (HSI) | Humectant |
| Hydrated silica | Abrasive |
| Sodium saccharin | Flavouring |
| Cellulose | Thickening agent |
| PEG-32 | Humectant |
| Xylitol | Humectant |
| Sodium fluoride | Anti-caries |
|  | agent |
| Flavouring | / |
| Oil of *Thymus vulgaris* leaves | Preservative |
| CI-77891 | Dye |
| PHB |  |

b) Production method of the toothpaste formulations.

The first step in the production method of each toothpaste formulation comprises the preparation of two distinct steps (conventionally identified as step A and step B) which at a later time were mixed together by means of a turbo-emulsifier (Silverson, UK). Table 6 shows the composition of each step.

TABLE 6

| Step A |
|---|
| Hydrogenated starch hydrolyzate (HSI) |
| Cellulose |
| Xylitol |
| **Step B** |
| Water |
| Sodium saccharin |
| Sodium fluoride |
| Zinc citrate |
| Strontium chloride |
| Papain |

After weighing all the ingredients, each step was brought to a temperature of 40°C in a thermostated bath. Once the desired temperature was reached and the ingredients had dissolved, the two steps were combined and homogenized with the aid of a turbo-emulsifier at 4000 rpm for a few minutes. At this point, by gradually increasing the speed of the instrument up to 4200 - 4300 rpm and taking care to perfectly incorporate the added amount before proceeding to the next aliquot, silica was added. The remaining ingredients were added to the so obtained emulsion, waiting for the complete homogenization of each ingredient before adding the next one. The PHB was the last element to be added.

c) Determination of the rheological properties of the toothpaste formulations.

An important characteristic to evaluate in a toothpaste formulation is its rheology, i.e. the study of its deformability following the application of an external force. The viscosity of a material decreases as the intensity of the force acting on it increases ("shear rate"). The deformability is predictive of the behaviour of the formulation once inserted into its sales packaging (usually a tube).

The rheological measurements were performed with a Kinexus rotational rheometer having a flat-cone geometry, using the parameters shown in Table 7. The measurements were carried out at a temperature of 25°C, with the same geometry, using a quantity of toothpaste in accordance with the needs of the instrument ("trimming").

TABLE 7

| Instrument parameters | |
|---|---|
| Temperature | 25°C |
| Start Shear Rate | 0.1000 s$^{-1}$ |
| End Shear Rate | 100.0 s$^{-1}$ |
| Samples per decade | 5 |

The analysed formulations showed a reduction in viscosity as the exerted force increased, i.e. as the shear rate increased.

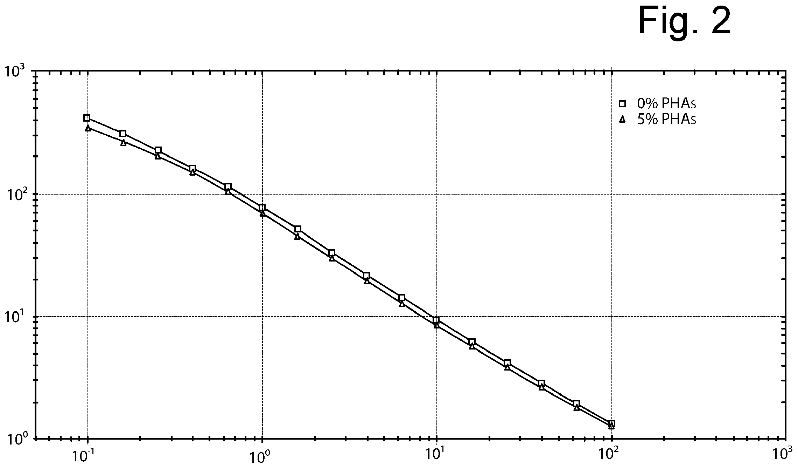

Figure 2 shows the viscosity curves as a function of the shear rate for the standard formulation containing 5% by weight of PHB, compared with the same formulation without the addition of PHB. As can be seen, the trends of the two curves are substantially overlapping, with a slight difference in favour of the formulation with PHB in terms of reduced viscosity with an increase in shear rate.

d) Evaluation of the toothpaste formulation's capacity to induce the coaggregation of bacteria on the PHB, subtracting them to adhesion to the dental surface.

The bacterial coaggregation analysis on PHB particles in toothpaste formulations was performed in triplicate on microorganisms attached to hydroxyapatite supports within sterile multi-well plates. The analyses were performed on the following strains: cariogenic microorganisms *S. mutans* ATCC 25175 and *S. sobrinus* ATCC 33478 and non-cariogenic microorganisms S. *salivarius* ATCC 13419 and *S. sanguis* ATCC 10556. For each bacterial strain, tests were carried out with two different adhesion times on the hydroxyapatite supports, i.e. after 2 hours and after 6 hours, using the following analysis criteria:

- incubation (37°C, 150 rpm) of the hydroxyapatite supports with a bacterial suspension obtained by diluting 1:10 a culture broth with optical density equal to 0.8 (600 nm) in *"Brain and Heart Infusion Broth"* (BHI Broth);
- post-incubation, each support was washed 3 times with a sterile buffered saline solution (PBS, pH 7.2), to remove the non-adherent bacterial cells, and was placed in contact, for 1 minute, with 1 mL of each 1:3 diluted toothpaste formulation in sterile distilled water;
- post-treatment, each support was washed 3 times with a sterile PBS solution and the microbial biomass adhering to the support was determined by carrying out a crystal-violet stain and reading the optical density (O.D.) at 595 nm. The optical density measurement is a parameter that is indicative of the bacterial load amount adhering to the surface of hydroxyapatite supports.

All the tests were performed using a toothpaste formulation without PHB as a control sample. This control made it possible to show that the coaggregation effect is due to the PHB and not to the other components of the toothpaste formulation.

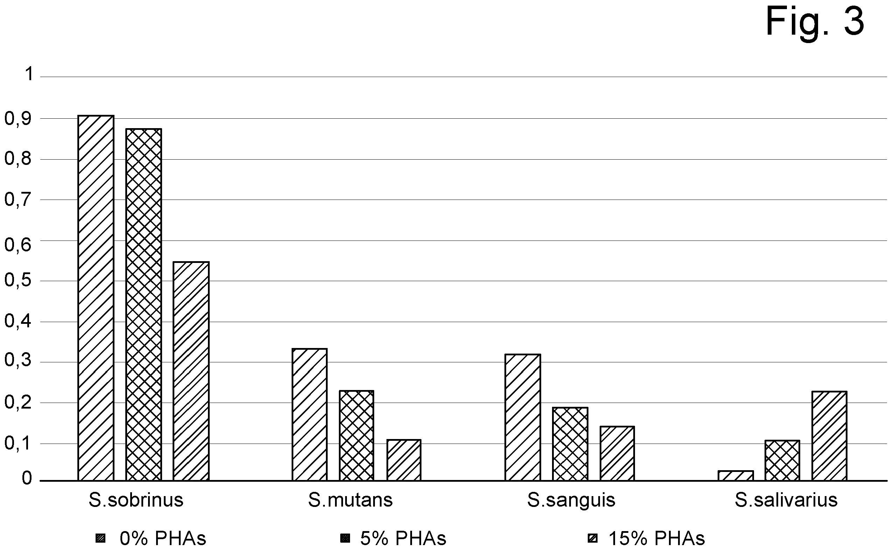

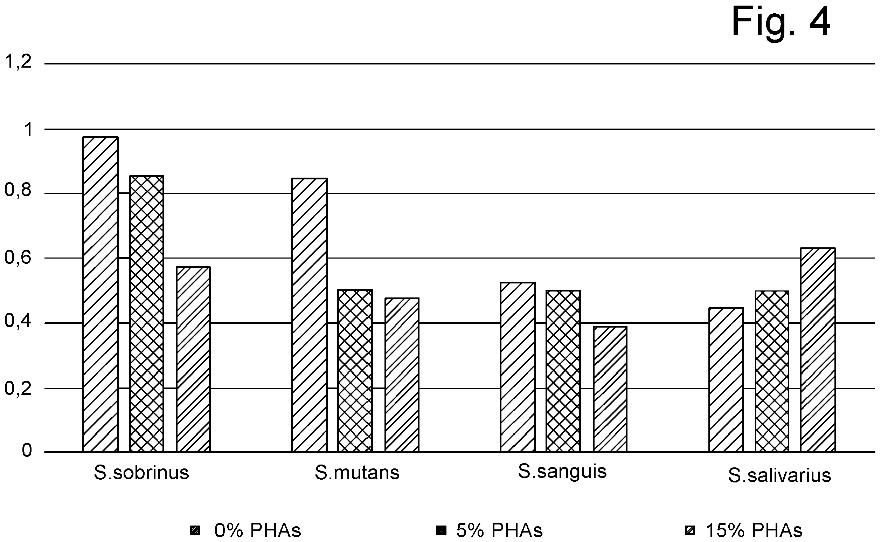

The four toothpaste formulations tested showed substantially identical results. Figures 3 and 4 show the coaggregating capacity of the standard formulation with and without PHB powder (5% and 15%) in terms of the four microbial strains adhering to the hydroxyapatite supports after 2 hours (Figure 2) and 6 hours (Figure 4).

For the two cariogenic strains, *S. mutans* and S. *sobrinus,* and for the strain *S. sanguis* (opportunistic pathogen), the tests showed that the treatment with a toothpaste formulation containing PHB caused a substantial removal of bacterial strains by the hydroxyapatite support.

This data clearly demonstrates that the toothpaste formulations comprising PHB according to the present invention are capable of sequestrating, and therefore removing, the bacterial strains adhering to the surface of the hydroxyapatite support, and therefore to the dental surface which, as is known, is rich in hydroxyapatite. This avoids the development of any pathological conditions deriving from the action of these bacteria inside the oral cavity.

**EXAMPLE 3: formulation of a chewing gum.**

a) A chewing gum formulation containing 10% PHB (the same used in the previous tests) was produced, having the composition indicated in Table 8.

TABLE 8

| Ingredient | Quantity (%) |
|---|---|
| Gum Base | 30 |
| Colophony | 20 |
| Cellulose | 18 |
| PHB | 10 |
| Sorbitol | 8 |
| Xylitol | 6 |
| Sunflower lecithin | 5 |
| Isomalt | 2.2 |
| Flavouring | 0.8 |

b) Description of the production method of the chewing gum formulation.

By maintaining a temperature of 90°C and using a mixer "Sigma Blade Mixer" (Ambica Boiler & Fabricator, India), gum base, cellulose and the PHB were mixed for 90 minutes. Subsequently, once the ingredients were amalgamated, colophony was added and the temperature was raised up to 120°C. The ingredients were again mixed until the formulation assumed a sufficiently smooth appearance, after which the latter was divided into smaller portions. Each portion was inserted inside a machinery "Logo Candy Disc Cutting Machine" (Loynds, UK) and flattened by rollers until sheets were obtained with a thickness of approximately 0.43 cm. Each sheet was then cut to form units, which were placed inside a special confectioner (or bassin - Loynds, UK) where they were coated with isomalt, thus obtaining chewing gum.

**Claims**

1. Formulation comprising at least one polyhydroxyalkanoate (PHA) and at least one physiologically acceptable excipient, for use in the treatment and/or prevention of diseases caused by pathogenic microorganisms of the oral cavity;

   wherein the PHA is polyhydroxybutyrate (PHB) in the form of particles with an average diameter (d50) from 0.1 um to 500 um;
   wherein the diseases caused by pathogenic microorganisms of the oral cavity are selected from: caries, gingivitis, periodontitis, perimplantitis, halitosis,
   wherein the formulation is selected from: toothpaste, chewing gum, mouthwash, dental paste, orodispersible tablet, gingival dye.

2. Formulation for use according to claim 1, wherein the PHA is in the form of particles with an average diameter (d50) from 0.5 um to 300 $\mu$m, preferably from 1 um to 100 um.

3. Formulation for use according to claim 1 or 2, wherein the PHA is present in a concentration comprised from 1% to 30%, preferably from 2% to 15% by weight, the % being expressed with respect to the overall weight of the formulation.

4. Formulation for use according to any of the claims 1 to 3, wherein the PHA is in the form of particles having a surface area value (BET) from 0.5 to 20 m$^2$/g, preferably from 1 to 10 m$^2$/g, measured according to norm ISO 9277:1995.

5. Formulation for use according to any of the claims 1 to 4, wherein the diseases are caused by pathogenic microorganisms or opportunistic pathogens selected from: *Streptococcus mutans, Streptococcus sobrinus, Streptococcus sanguis, Porfiromonas gingivalis, Aggregatibacter actinomycetemcomitans,* or fungi such as *Aspergillus fumigatus, Aspergillus flavus* and *Candida albicans.*

**Patentansprüche**

1. Formulierung, umfassend mindestens ein Polyhydroxyalkanoat (PHA) und mindestens einen physiologisch verträglichen Hilfsstoff, zur Verwendung in der Behandlung und/oder Vorbeugung von Erkrankungen, die durch pathogene Mikroorganismen der Mundhöhle verursacht werden;

   wobei das PHA Polyhydroxybutyrat (PHB) in Form von Partikeln mit einem durchschnittlichen Durchmesser (d50) von 0,1 μm bis 500 μm ist;
   wobei die durch pathogene Mikroorganismen der Mundhöhle verursachten Erkrankungen ausgewählt sind aus: Karies, Gingivitis, Parodontitis, Perimplantitis, Halitosis,
   wobei die Formulierung ausgewählt ist aus: Zahnpasta, Kaugummi, Mundwasser, Dental-Pasta, Schmelztablette, Zahnfleischfarbstoff.

2. Formulierung zur Verwendung nach Anspruch 1, wobei das PHA in Form von Partikeln mit einem durchschnittlichen Durchmesser (d50) von 0,5 μm bis 300 μm, vorzugsweise von 1 μm bis 100 μm, vorliegt.

3. Formulierung zur Verwendung nach Anspruch 1 oder 2, wobei das PHA in einer Konzentration von 15 bis 30 Gew.-%, vorzugsweise von 2 bis 15 Gew.-% vorliegt, wobei 95 in Bezug auf das Gesamtgewicht der Formulierung ausgedrückt wird.

4. Formulierung zur Verwendung nach einem der Ansprüche 1 bis 3, wobei das PHA in Form von Partikeln mit einem Oberflächenwert (BET) von 0,5 bis 20 m$^2$/g, vorzugsweise von 1 bis 10 m$^2$/g, gemessen gemäß der Norm ISO 9277:1995, vorliegt.

5. Formulierung zur Verwendung nach einem der Ansprüche 1 bis 4, wobei die Erkrankungen durch pathogene Mikroorganismen oder opportunistische Krankheitserreger verursacht werden, ausgewählt aus: *Streptococcus mutans, Streptococcus sobrinus, Streptococcus sanguis, Porfiromonas gingivalis, Aggregatibacter actinomycetemcomitans,* oder Pilze wie *Aspergillus fumigatus, Aspergillus flavus* und *Candida albicans.*

**Revendications**

1. Formulation comprenant au moins un polyhydroxyalcanoate (PHA) et au moins un excipient physiologiquement acceptable, à utiliser dans le traitement et/ou la prévention de maladies causées par des micro-organismes pathogènes de la cavité buccale;

   dans laquelle le PHA est le polyhydroxybutyrate (PHB) sous forme de particules dont le diamètre moyen (d50) est compris entre 0,1 μm et 500 μm;
   dans laquelle les maladies causées par des micro-organismes pathogènes de la cavité buccale sont choisies parmi : les caries, la gingivite, la parodontite, la péri-implantite, l'halitose,
   dans laquelle la formulation est choisie parmi : le dentifrice, le chewing-gum, le bain de bouche, la pâte dentaire, le comprimé orodispersible, le colorant gingival.

2. Formulation à utiliser selon la revendication 1, dans laquelle le PHA se présente sous la forme de particules dont le diamètre moyen (d50) est compris entre 0,5 μm et 300 μm, de préférence entre 1 μm et 100 μm.

3. Formulation à utiliser selon la revendication 1 ou 2, dans laquelle le PHA est présent dans une concentration comprise entre 15 et 30%, de préférence entre 2% et 15% en poids, le 95 étant exprimé par rapport au poids total de la formulation.

4. Formulation à utiliser selon l'une quelconque des revendications 1 à 3, dans laquelle le PHA est sous forme de particules ayant une valeur de surface (BET) de 0,5 à 20 m$^2$/g, de préférence de 1 à 10 m$^2$/g, mesurée selon la norme ISO 9277:1995.

5. Formulation à utiliser selon l'une quelconque des revendications 1 à 4, dans laquelle les maladies sont causées par des micro-organismes pathogènes ou des agents pathogènes opportunistes choisis parmi: *Streptococcus mutans, Streptococcus sobrinus, Streptococcus sanguis, Porfiromonas gingivalis, Aggregatibacter actinomycetemcomitans,* ou des champignons tels que *Aspergillus fumigatus, Aspergillus flavus* et *Candida albicans.*

Fig. 1

# Fig. 2

EP 3 897 674 B1

Fig. 3

Fig. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007146173 A
- WO 9923146 A
- WO 2011045625 A
- WO 2015015315 A
- US 8956835 B2

**Non-patent literature cited in the description**

- A Guidebook to Particle Size Analysis. Horiba Instruments Inc, 2016